# EUROPEAN PATENT APPLICATION

(11) **EP 4 785 947 A1**
(43) Date of publication of application: **05.08.2026**
(21) Application number: 25155241.0
(22) Date of filing: 31.01.2025
(51) Int. Cl.: A61K 36/185, A61P 9/14, A61K 9/00, A61K 9/48, A61K 36/29, A61K 36/58, A61K 36/77, A61K 36/87, A61K 36/28

(54) **HERBAL MEDICINE FOR THE TREATMENT OF HEMORRHOIDAL DISEASES**

(71) Applicant: Terra Lab International D.O.O., 10000 Zagreb (HR)
(72) Inventor: KARLO, Alen, 10000 Zagreb (HR)
(74) Representative: Maiwald GmbH

(57) **Abstract**

The present disclosure is directed to three aspects, a pharmaceutical composition for use in treating hemorrhoidal disease and/ or the associated symptoms of hemorrhoidal disease, a method of producing said composition and a capsule comprising said composition. The pharmaceutical composition contains seven different herbal extracts of plant material of cichorium intybus, vitis vinifera, berberis aristate, hamamelis virginiana, azadirachta indica, aesculus hippocastanum and mesua ferrea that surprisingly alleviates symptoms of hemorrhoids and/or hemorrhoidal disease, even in cases where symptoms persist despite prior treatment with conventional local or oral medicinal therapies.

## Description

### Technical Field

The present disclosure relates to a pharmaceutical composition to treat hemorrhoidal diseases and/or the symptoms associated with hemorrhoidal diseases.

### Background

Hemorrhoidal disease is a common anal condition, defined as the symptomatic enlargement and distal displacement of the normal anal cushions. Hemorrhoidal disease is one of the most common causes of anal pathology, with a peak incidence between the ages of 45 and 65 years in both males and females.

Hemorrhoidal disease is a prevalent anorectal condition characterized by symptoms such as pain, discomfort, itching, swelling, and rectal bleeding. The pathophysiology of hemorrhoidal disease remains incompletely understood, but the sliding of the anal canal lining and the deterioration of supporting tissues in the anal cushions are recognized as major contributing factors. Patients with hemorrhoidal disease exhibit significant pathological changes, including venous dilation, vascular thrombosis, and degeneration of collagen fibers and fibroelastic tissues. Additionally, distortion and rupture of the anal subepithelial muscle, severe inflammatory reactions involving the vascular wall, and mucosal ulceration with ischemia and thrombosis are commonly observed in hemorrhoidal specimens.

Symptomatically, hemorrhoidal disease causes pain and discomfort in a majority of patients, with 89.4 % and 94.9 % of individuals, respectively, reporting these issues. Other common symptoms include itching (60.8 %), swelling (60.6 %), and bleeding (57.9 %). Soiling is less frequently reported, occurring in 24.9 % of cases. The Goligher classification system is commonly used to grade disease severity, with most patients presenting with grade I (29.3 %) or grade II (42.8 %) hemorrhoids at diagnosis. Hemorrhoidal disease is also associated with a high recurrence rate, with 48.4 % of treated patients reporting a prior history of the condition.

Factors contributing to hemorrhoidal disease development are often linked to increased intraabdominal pressure, such as constipation, straining during defecation, pregnancy, and obesity, although clear causal relationships remain unproven. Depending on the severity of hemorrhoidal disease, treatment options range from lifestyle modifications (e.g., dietary changes, regular exercise, and avoidance of straining) to non-invasive medical therapies, minimally invasive procedures (e.g., sclerotherapy, rubber band ligation, infrared coagulation, and cryotherapy), and surgical interventions (e.g., hemorrhoidectomy, hemorrhoidal artery ligation, and stapled hemorrhoidopexy).

The primary goal of medical treatments for hemorrhoidal disease is to manage acute symptoms rather than cure the underlying condition. Among the available treatments, phlebotonics, particularly flavonoid-based compounds, are widely used oral medications. These agents, derived from plant extracts or synthesized compounds such as calcium dobesilate, enhance vascular tone, improve lymphatic drainage, reduce vascular capacity, and stabilize capillary permeability. Despite their widespread use and demonstrated benefits in alleviating bleeding, pruritus, and other symptoms, the precise mechanism of action of phlebotonics remains unclear.

However, a significant subset of patients experiences solely minimal, transient or even no improvement with existing medical therapies and seeks non-invasive or adjunct treatments that are both effective and safe. This unmet need underscores the importance of developing a novel medicine that provides comprehensive and sustained symptom relief for individuals suffering from hemorrhoidal disease.

### Summary

In a first aspect, the present disclosure is directed to a pharmaceutical composition for use in treating hemorrhoidal disease and/ or the associated symptoms of hemorrhoidal disease, wherein the composition for use in treating a hemorrhoidal disease and/or the symptoms associated with a hemorrhoidal disease, wherein the composition comprises:
a dry extract of the root of cichorium intybus,
a dry extract of the leaves of vitis vinifera,
a dry extract of the root and bark of berberis aristate,
a dry extract of the bark of hamamelis virginiana,
a dry extract of the leaves of azadirachta indica,
a dry extract of the seeds of aesculus hippocastanum,
a dry extract of flower material of mesua ferrea.

The inventors of the present disclosure surprisingly found that pharmaceutical composition according to the first aspect enables improvement or resolution of hemorrhoidal disease and its symptoms.

In a second aspect, the present disclosure is directed to a method of producing a pharmaceutical composition according to any one of the preceding claims, wherein the method comprises the steps of:
i. extracting each plant material of cichorium intybus, vitis vinifera, berberis aristate, hamamelis virginiana, azadirachta indica, aesculus hippocastanum and mesua ferrea with an aqueous or hydroalcoholic solvent;
ii. drying of each extract to obtain a powder extract;
iii. providing corresponding amounts of each dried powder extract to obtain the pharmaceutical composition;
iv. optionally blending the pharmaceutical composition, and optionally adding fillers and/or excipients to the pharmaceutical composition prior to blending, to obtain a uniformly distributed powder blend of the pharmaceutical composition;

In a third aspect, the present disclosure relates to a capsule comprising the pharmaceutical composition for use according to the first aspect, wherein the capsule comprises 118.5 to 760 mg of the pharmaceutical composition.

Unexpectedly, with rapid onset of effect, the pharmaceutical composition of the present disclosure demonstrats benefit regardless of the symptom's combination present in patients and throughout disease severity (grade I - III). Moreover, the composition according to the present disclosure has been efficient in patients with persisting symptoms and more importantly also after failure of local or oral medicinal therapy and their combination.

### Detailed Description

The aspects of the present disclosure are described in the following in more detail, exemplified by preferred embodiments and embodiment examples. However, it is understood that the scope of the present disclosure is not limited thereto, but only by the appendant claims.

### The pharmaceutical composition

In a first aspect, the present disclosure is directed to a pharmaceutical composition for use in treating hemorrhoidal disease and/ or the associated symptoms of hemorrhoidal disease, wherein the composition for use in treating a hemorrhoidal disease and/or the symptoms associated with a hemorrhoidal disease, wherein the composition comprises:
a dry extract of the root of cichorium intybus,
a dry extract of the leaves of vitis vinifera,
a dry extract of the root and bark of berberis aristate,
a dry extract of the bark of hamamelis virginiana,
a dry extract of the leaves of azadirachta indica,
a dry extract of the seeds of aesculus hippocastanum,
a dry extract of flower material of mesua ferrea.

The pharmaceutical composition of the present disclosure contains seven different dry extracts from the plants cichorium intybus, vitis vinifera, berberis aristate, hamamelis virginiana, azadirachta indica, aesculus hippocastanum, and mesua ferrea, as listed above, which together are effective against hemorrhoidal disease and/or its symptoms. In particular, the effect results from an amount of active ingredient in the respective plant extract.

In a preferred embodiment the pharmaceutical composition for use according to the present disclosure is effective against hemorrhoidal disease and/or its symptoms, wherein the symptoms associated with the hemorrhoidal disease are selected from the group consisting of itching in the anal region, irritation in the anal region, swelling near the anus, inflammation, mucosal ulceration, ischemia, thrombosis, external hemorrhoids, rectal bleeding, protrusion, pain and discomfort.

The pharmaceutical composition for use according to the present disclosure, composed of seven different herbal extracts, has been found to surprisingly alleviate symptoms of hemorrhoids and hemorrhoidal disease, even in cases where symptoms persist despite prior treatment with conventional local or oral medicinal therapies.

Unlike standard treatments that often provide only temporary relief, this formulation demonstrates efficacy across all grades of hemorrhoids, from mild discomfort (Grade I) to severe prolapsed hemorrhoids (Grade III). Its multi-targeted mechanism addresses inflammation, vascular integrity, and tissue healing, providing relief from pain, itching, swelling, and bleeding. Notably, the formulation has shown therapeutic benefits even in patients who have experienced inadequate response to standard therapies, making it a promising option for those suffering from chronic or treatment-resistant hemorrhoidal disease.

In the treatment of a hemorrhoidal disease, suitable administration methods for a pharmaceutical composition include topical application, rectal administration, and systemic delivery. Topical formulations, such as creams, ointments, or gels, are directly applied to the affected area to provide localized relief from symptoms like pain, itching, and inflammation. Rectal administration, including suppositories or enemas, ensures targeted delivery to internal hemorrhoids, promoting effective treatment and minimizing systemic absorption. For severe or refractory cases, systemic delivery via oral or injectable formulations may be considered to address underlying causes and provide anti-inflammatory or analgesic effects.

In a preferred embodiment, the pharmaceutical composition according to the present disclosure is administered by oral administration.

For oral administration, suitable forms may include any type of powder formulation, such as tablets, pills, pellets, granules, and powder containing capsules. Tablets, any sort of compressed powder dose and capsules are preferred for their precise dosing, convenience, and stability. Controlled-release or enteric-coated tablets can provide sustained delivery of active ingredients, reducing the frequency of administration and enhancing patient compliance.

In a preferred embodiment, the pharmaceutical composition for use according to the present disclosure can be in the form of tablets, pills, pellets, powders, granules, capsules, capsules containing powders, or any other form suitable for use, preferably wherein composition is in the form of a tablet or of a capsule.

Furthermore, the pharmaceutical composition for use is made up of different amounts of seven herbal components, which are administered in a specific daily dosage:

### Cichorium intybus

Cichorium intybus, commonly known as chicory, is a hardy perennial plant of the Asteraceae family, cultivated for its culinary and medicinal applications.

A dry extract form of cichorium intybus is a concentrated powder derived from its roots, leaves, or whole plant through a process of solvent extraction and drying. According to the present disclosure, the dry extract form of cichorium intybus is a concentrated powder extracted from the root (radix) of cichorium intybus.

In a preferred embodiment, the pharmaceutical composition for use according to the present disclosure comprises a dry extract of the root of cichorium intybus in an amount of from 30 to 90 mg.

In a further preferred embodiment, the pharmaceutical composition for use comprises a dry extract of the root of cichorium intybus in an amount of from 45 to 70 mg.

In another further preferred embodiment, the pharmaceutical composition for use comprises a dry extract of the root of cichorium intybus in an amount of from 55 to 60 mg.

In another further preferred embodiment, the pharmaceutical composition for use comprises a dry extract of the root of cichorium intybus in an amount of 60 mg.

The dry extract of cichorium intybus retains bioactive compounds, such as inulin, sesquiterpene lactones, polyphenols, and flavonoids, that confer its therapeutic properties. Especially, its root (radix) is processed to produce inulin.

Inulin is a naturally occurring polysaccharide and dietary fiber. Composed primarily of fructose units, inulin serves as a prebiotic, promoting the growth of beneficial gut bacteria. Its non-digestible nature allows it to pass through the gastrointestinal tract, aiding in improved digestion, enhanced mineral absorption, and better gut health.

In a preferred embodiment, the pharmaceutical composition for use according to the present disclosure comprises the dry extract of the root of cichorium intybus, wherein the dry extract of the root of cichorium intybus contains 45 wt.-% to 95 wt.-% of inulin as active agent.

In a further preferred embodiment, the pharmaceutical composition for use according to the present disclosure comprises the dry extract of the root of cichorium intybus, wherein the dry extract of the root of cichorium intybus contains 60 wt.-% to 90 wt.-% of inulin as active agent. In other words, 60 wt.-% to 90 wt.-% of the dry extract consist of inulin.

In another further preferred embodiment, the pharmaceutical composition for use according to the present disclosure comprises the dry extract of the root of cichorium intybus, wherein the dry extract of the root of cichorium intybus contains 90 wt.-% of inulin as active agent.

All preferred quantities of extract of the root of cichorium intybus in an amount of from 30 to 90 mg may have a certain amount of active ingredient inulin between 45 wt.-% to 95 wt.-%.

In a preferred embodiment, the pharmaceutical composition for use comprises a dry extract of the root of cichorium intybus in an amount of 60 mg, wherein the dry extract contains 45 wt.-% to 95 wt.-% of inulin as active agent.

In a further preferred embodiment, the pharmaceutical composition for use comprises a dry extract of the root of cichorium intybus in an amount of 60 mg, wherein the dry extract contains 60 wt.-% to 90 wt.-% of inulin as active agent.

In another further preferred embodiment, the pharmaceutical composition for use comprises a dry extract of the root of cichorium intybus in an amount of 60 mg, wherein the dry extract contains 90 wt.-% of inulin as active agent.

### Vitis vinifera

Vitis vinifera is commonly known as the grapevine and is a widely cultivated plant species renowned for its use in winemaking, table grapes, and raisins.

A dry extract form of vitis vinifera is a concentrated powder extracted from the seeds, skins (of the grapes), and/or leaves of the grapevine, through a process of solvent extraction and drying. According to the present disclosure, the dry extract form of vitis vinifera is a concentrated powder extracted from the leaves of vitis vinifera.

In a preferred embodiment, the pharmaceutical composition for use according to the present disclosure comprises a dry extract of the leaves of vitis vinifera in an amount of from 40 to 200 mg.

In a further preferred embodiment, the pharmaceutical composition for use comprises a dry extract of the leaves of vitis vinifera in an amount of from 80 to 150 mg.

In another further preferred embodiment, the pharmaceutical composition for use comprises a dry extract of the leaves of vitis vinifera in an amount of from 100 to 130 mg.

In another further preferred embodiment, the pharmaceutical composition for use comprises a dry extract of the leaves of vitis vinifera in an amount of 120 mg.

Vitis vinifera contains bioactive compounds, including resveratrol, flavonoids, and proanthocyanidins, found primarily in its seeds, skin, and leaves. These compounds exhibit potent antioxidant, anti-inflammatory, and cardioprotective properties, making vitis vinifera extracts valuable in nutraceuticals, cosmetics, and pharmaceutical formulations.

In a preferred embodiment, the pharmaceutical composition for use according to the present disclosure comprises the dry extract of the leaves of vitis vinifera, wherein the dry extract of the leaves of vitis vinifera contains 30 wt.-% to 95 wt.-% of oligomeric proanthocyanidins as active agent.

Oligomeric proanthocyanidins (OPCs) are a group of potent polyphenolic compounds found in various plants, particularly among grape seeds (Vitis vinifera), and also in pine bark, and berries. Renowned for their strong antioxidant properties, OPCs neutralize free radicals, reducing oxidative stress and supporting cellular health. They are associated with numerous health benefits, including improved anti-inflammatory effects.

In a further preferred embodiment, the pharmaceutical composition for use according to the present disclosure comprises the dry extract of the leaves of vitis vinifera, wherein the dry extract of the leaves of vitis vinifera contains 60 wt.-% to 95 wt.-% of oligomeric proanthocyanidins as active agent.

In another further preferred embodiment, the pharmaceutical composition for use according to the present disclosure comprises the dry extract of the leaves of vitis vinifera, wherein the dry extract of the leaves of vitis vinifera contains 95 wt.-% of oligomeric proanthocyanidins as active agent.

All preferred quantities of extract of the leaves of vitis vinifera in an amount of from 40 to 200 mg may have a certain amount of active ingredients oligomeric proanthocyanidins between 30 wt.-% to 95 wt.-%.

In a preferred embodiment, the pharmaceutical composition for use comprises a dry extract of the leaves of vitis vinifera in an amount of 120 mg, wherein the dry extract contains 30 wt.-% to 95 wt.-% of oligomeric proanthocyanidins as active agent.

In a further preferred embodiment, the pharmaceutical composition for use comprises a dry extract of the leaves of vitis vinifera in an amount of 120 mg, wherein the dry extract contains 60 wt.-% to 95 wt.-% of oligomeric proanthocyanidins as active agent.

In another further preferred embodiment, the pharmaceutical composition for use comprises a dry extract of the leaves of vitis vinifera in an amount of 120 mg, wherein the dry extract contains 95 wt.-% of oligomeric proanthocyanidins as active agent.

### Berberis aristate

Berberis aristate is commonly known as Indian barberry or tree turmeric and is a medicinal plant native to the Himalayan region and valued for its therapeutic properties.

A dry extract of berberis aristate is a concentrated powder derived from the roots or bark of the plant, through a process of solvent extraction and drying. According to the present disclosure, the dry extract form of berberis aristate is a concentrated powder extracted from the roots and bark of berberis aristate.

In a preferred embodiment, the pharmaceutical composition for use according to the present disclosure comprises a dry extract of the root and bark of berberis aristate in an amount of from 65 to 500 mg.

In a further preferred embodiment, the pharmaceutical composition for use comprises a dry extract of the root and bark of berberis aristate in an amount of from 150 to 400 mg.

In another further preferred embodiment, the pharmaceutical composition for use comprises a dry extract of the root and bark of berberis aristate in an amount of from 280 to 350 mg.

In another further preferred embodiment, the pharmaceutical composition for use comprises a dry extract of the root and bark of berberis aristate in an amount of 320 mg.

The extract of berberis aristate contains bioactive compounds to which potent pharmacological properties, including antimicrobial, anti-inflammatory, and hypoglycemic effects are attributed. The plant's key bioactive component is the naturally occurring alkaloid berberine that has antimicrobial, anti-inflammatory, and antidiabetic effects.

In a preferred embodiment, the pharmaceutical composition for use according to the present disclosure comprises the dry extract of the root and bark of berberis aristate, wherein the dry extract of the root and bark of berberis aristate contains 0.4 wt.-% to 7 wt.-% of berberine as active agent.

In a further preferred embodiment, the pharmaceutical composition for use according to the present disclosure comprises the dry extract of the root and bark of berberis aristate, wherein the dry extract of the root and bark of berberis aristate contains 1 wt.-% to 3 wt.-% of berberine as active agent.

In another further preferred embodiment, the pharmaceutical composition for use according to the present disclosure comprises the dry extract of the root and bark of berberis aristate, wherein the dry extract of the root and bark of berberis aristate contains 2 wt.-% of berberine as active agent.

All preferred quantities of extract of the root and bark of berberis aristate in an amount of from 65 to 500 mg may have a certain amount of active ingredient berberine between 0.4 wt.-% to 7 wt.-%.

In a preferred embodiment, the pharmaceutical composition for use comprises a dry extract of the root and bark of berberis aristate in an amount of 320 mg, wherein the dry extract contains 0.4 wt.-% to 7 wt.-% of berberine as active agent.

In a further preferred embodiment, the pharmaceutical composition for use comprises a dry extract of the root and bark of berberis aristate in an amount of 320 mg, wherein the dry extract contains 1 wt.-% to 3 wt.-% of berberine as active agent.

In another further preferred embodiment, the pharmaceutical composition for use comprises a dry extract of the root and bark of berberis aristate in an amount of 320 mg, wherein the dry extract contains 2 wt.-% of berberine as active agent.

### Hamamelis virginiana

Hamamelis virginiana is commonly known as witch hazel and is a plant recognized for its astringent, anti-inflammatory, and antioxidant properties.

A dry extract of hamamelis virginiana is a concentrated powder typically derived from dried leaves and/or bark of the plant, through a process of solvent extraction and drying. Hamamelis virginiana extracts are frequently incorporated into compositions intended for dermatological use, including creams, gels, and lotions, for the treatment of skin irritation, wounds, and varicose veins. According to the present disclosure, the dry extract form of hamamelis virginiana is a concentrated powder extracted from the bark of hamamelis virginiana.

In a preferred embodiment, the pharmaceutical composition for use according to the present disclosure comprises a dry extract of the bark of hamamelis virginiana in an amount of from 25 to 130 mg.

In a further preferred embodiment, the pharmaceutical composition for use comprises a dry extract of the bark of hamamelis virginiana in an amount of from 50 to 110 mg.

In another further preferred embodiment, the pharmaceutical composition for use comprises a dry extract of the bark of hamamelis virginiana in an amount of from 70 to 90 mg.

In another further preferred embodiment, the pharmaceutical composition for use comprises a dry extract of the bark of hamamelis virginiana in an amount of 80 mg.

Extracts derived from hamamelis virginiana bark contain bioactive compounds such as tannins, flavonoids, and polyphenols, which contribute to its therapeutic effects. Water-soluble tannins, a subclass of polyphenolic compounds, exhibit significant pharmaceutical benefits due to their astringent, anti-inflammatory, antioxidant, and antimicrobial properties. Due to their water solubility, hydrolysable tannins demonstrate improved bioavailability, allowing for efficient incorporation into oral or topical pharmaceutical compositions.

In a preferred embodiment, the pharmaceutical composition for use according to the present disclosure comprises the dry extract of the bark of hamamelis virginiana, wherein the dry extract of the bark of hamamelis virginiana contains 2 wt.-% to 10 wt.-% of hydrolysable tannins as active agent.

In a further preferred embodiment, the pharmaceutical composition for use according to the present disclosure comprises the dry extract of the bark of hamamelis virginiana, wherein the dry extract of the bark of hamamelis virginiana contains 7 wt.-% to 10 wt.-% of hydrolysable tannins as active agent.

In another further preferred embodiment, the pharmaceutical composition for use according to the present disclosure comprises the dry extract of the bark of hamamelis virginiana, wherein the dry extract of the bark of hamamelis virginiana contains 10 wt.-% of hydrolysable tannins as active agent.

All preferred quantities of extract of the bark of hamamelis virginiana in an amount of from 25 to 130 mg may have a certain amount of active ingredient hydrolysable tannins between 2 wt.-% to 10 wt.-%.

In a preferred embodiment, the pharmaceutical composition for use comprises a dry extract of the bark of hamamelis virginiana in an amount of 80 mg, wherein the dry extract contains 2 wt.-% to 10 wt.-% of hydrolysable tannins as active agent.

In a further preferred embodiment, the pharmaceutical composition for use comprises a dry extract of the bark of hamamelis virginiana in an amount of 80 mg, wherein the dry extract contains 7 wt.-% to 10 wt.-% of hydrolysable tannins as active agent.

In another further preferred embodiment, the pharmaceutical composition for use comprises a dry extract of the bark of hamamelis virginiana in an amount of 80 mg, wherein the dry extract contains 10 wt.-% of hydrolysable tannins as active agent.

### Azadirachta indica

Azadirachta indica is commonly known as neem and is a medicinal plant that is recognized for its antibacterial, antifungal, anti-inflammatory, and immunomodulatory properties.

A dry extract of azadirachta indica is a concentrated powder typically derived from dried parts of the plant, such as the leaves, the seeds, the bark, the flowers or the root. The powered extract of azadirachta indica can be obtained through a process of solvent extraction and drying. According to the present disclosure, the dry extract form of azadirachta indica is a concentrated powder extracted from the leaves of azadirachta indica.

In a preferred embodiment, the pharmaceutical composition for use according to the present disclosure comprises a dry extract of the leaves of azadirachta indica in an amount of from 4 to 35 mg.

In a further preferred embodiment, the pharmaceutical composition for use comprises a dry extract of the leaves of azadirachta indica in an amount of from 10 to 30 mg.

In another further preferred embodiment, the pharmaceutical composition for use comprises a dry extract of the leaves of azadirachta indica in an amount of from 15 to 25 mg.

In another further preferred embodiment, the pharmaceutical composition for use comprises a dry extract of the leaves of azadirachta indica in an amount of 20 mg.

The extract of leaves from azadirachta indica is a rich source of bioactive compounds, including limonoids, flavonoids, tannins, and polyphenols, which contribute to its broad-spectrum therapeutic properties. Among these, nimbidin is one of the most significant bioactive constituents, primarily isolated from neem seed oil. Nimbidin is a bitter triterpenoid known for its potent anti-inflammatory, antibacterial, antifungal, and immunomodulatory effects, making it valuable for pharmaceutical applications in dermatology, oral health, and gastrointestinal treatments.

In a preferred embodiment, the pharmaceutical composition for use according to the present disclosure comprises the dry extract of the leaves of azadirachta indica, wherein the dry extract of the leaves of azadirachta indica contains 0.2 wt.-% to 1 wt.-% of nimbidin as active agent.

In a further preferred embodiment, the pharmaceutical composition for use according to the present disclosure comprises the dry extract of the leaves of azadirachta indica, wherein the dry extract of the leaves of azadirachta indica contains 0.5 wt.-% to 1 wt.-% of nimbidin as active agent.

In another further preferred embodiment, the pharmaceutical composition for use according to the present disclosure comprises the dry extract of the leaves of azadirachta indica, wherein the dry extract of the leaves of azadirachta indica contains 1 wt.-% of nimbidin as active agent.

All preferred quantities of extract of the leaves of azadirachta indica in an amount of from 4 to 35 mg may have a certain amount of active ingredient nimbidin between 0.2 wt.-% to 1 wt.-%.

In a preferred embodiment, the pharmaceutical composition for use comprises a dry extract of the leaves of azadirachta indica in an amount of 20 mg, wherein the dry extract contains 0.2 wt.-% to 1 wt.-% of nimbidin as active agent.

In a further preferred embodiment, the pharmaceutical composition for use comprises a dry extract of the leaves of azadirachta indica in an amount of 20 mg, wherein the dry extract contains 0.5 wt.-% to 1 wt.-% of nimbidin as active agent.

In another further preferred embodiment, the pharmaceutical composition for use comprises a dry extract of the leaves of azadirachta indica in an amount of 20 mg, wherein the dry extract contains 1 wt.-% of nimbidin as active agent.

### Aesculus hippocastanum

Aesculus hippocastanum is commonly known as horse chestnut and is known as a medicinal plant used in pharmaceutical compositions for its veno-tonic, anti-inflammatory, and antioxidant properties.

Extracts of aesculus hippocastanum are primarily obtained from specific plant parts that contain high concentrations of biologically active compounds. The seeds are the most used source of plant material for extraction, rich in escin, flavonoids, and coumarins. The bark as well as the leaves contain tannins, coumarins and additional bioactive compounds, including flavonoids and phenolic acids. The powered extract can be obtained through a process of solvent extraction and drying. According to the present disclosure, the dry extract form of aesculus hippocastanum is a concentrated powder extracted from the seeds of aesculus hippocastanum.

In a preferred embodiment, the pharmaceutical composition for use according to the present disclosure comprises a dry extract of the seeds of aesculus hippocastanum in an amount of from 15 to 110 mg.

In a further preferred embodiment, the pharmaceutical composition for use comprises a dry extract of the seeds of aesculus hippocastanum in an amount of from 40 to 80 mg.

In another further preferred embodiment, the pharmaceutical composition for use comprises a dry extract of the seeds of aesculus hippocastanum in an amount of from 50 to 70 mg.

In another further preferred embodiment, the pharmaceutical composition for use comprises a dry extract of the seeds of aesculus hippocastanum in an amount of 60 mg.

Escin is the primary bioactive compound derived from seeds of aesculus hippocastanum and is a mixture of triterpenoid saponins known for its veno-tonic, anti-inflammatory, and anti-edematous properties. Escin strengthens capillary walls, reduces vascular permeability, and improves blood circulation and exhibits antioxidant and anti-inflammatory effects by reducing inflammatory mediators and protecting endothelial integrity.

In a preferred embodiment, the pharmaceutical composition for use according to the present disclosure comprises the dry extract of the seeds of aesculus hippocastanum, wherein the dry extract of the seeds of aesculus hippocastanum contains 5 wt.-% to 20 wt.-% of escin as active agent.

In a further preferred embodiment, the pharmaceutical composition for use according to the present disclosure comprises the dry extract of the seeds of aesculus hippocastanum, wherein the dry extract of the seeds of aesculus hippocastanum contains 10 wt.-% to 20 wt.-% of escin as active agent.

In another further preferred embodiment, the pharmaceutical composition for use according to the present disclosure comprises the dry extract of the seeds of aesculus hippocastanum, wherein the dry extract of the seeds of aesculus hippocastanum contains 20 wt.-% of escin as active agent.

All preferred quantities of extract of the seeds of aesculus hippocastanum in an amount of from 15 to 110 mg may have a certain amount of active ingredient escin between 5 wt.-% to 20 wt.-%.

In a preferred embodiment, the pharmaceutical composition for use comprises a dry extract of the seeds of aesculus hippocastanum in an amount of 60 mg, wherein the dry extract contains 5 wt.-% to 20 wt.-% of escin as active agent.

In a further preferred embodiment, the pharmaceutical composition for use comprises a dry extract of the seeds of aesculus hippocastanum in an amount of 60 mg, wherein the dry extract contains 10 wt.-% to 20 wt.-% of escin as active agent.

In another further preferred embodiment, the pharmaceutical composition for use comprises a dry extract of the seeds of aesculus hippocastanum in an amount of 60 mg, wherein the dry extract contains 20 wt.-% of escin as active agent.

### Mesua ferrea

Mesua ferrea is commonly known as the Indian rose chestnut or cobra saffron and is a medicinal plant valued for its anti-inflammatory, antimicrobial, antioxidant, and analgesic properties.

A dry extract of mesua ferrea is a concentrated powder typically derived from dried parts of the plant, such as from its flowers, seeds, bark, and leaves that include bioactive compounds. The powered extract is obtained through a process of solvent extraction and drying. According to the present disclosure, the dry extract form of mesua ferrea is a concentrated powder extracted from the flower of mesua ferrea.

In a preferred embodiment, the pharmaceutical composition for use according to the present disclosure comprises a dry extract of flower material of mesua ferrea in an amount of from 60 to 450 mg.

In a further preferred embodiment, the pharmaceutical composition for use comprises a dry extract of flower material of mesua ferrea in an amount of from 100 to 350 mg.

In another further preferred embodiment, the pharmaceutical composition for use comprises a dry extract of flower material of mesua ferrea in an amount of from 200 to 280 mg.

In another further preferred embodiment, the pharmaceutical composition for use comprises a dry extract of flower material of mesua ferrea in an amount of 240 mg.

Extracts of mesua ferrea contain several biological active compounds such as flavonoids, xanthones, tannins, and essential oils, which contribute to its pharmacological effects. Mesua ferrea is a valuable source of gallotannin, a hydrolysable tannin known for its antioxidant, anti-inflammatory, antimicrobial, and astringent properties.

In a preferred embodiment, the pharmaceutical composition for use according to the present disclosure comprises the dry extract of flower material of mesua ferrea, wherein the dry extract of flower material of mesua ferrea contains 3 wt.-% to 20 wt.-% of gallotannin as active agent.

In a further preferred embodiment, the pharmaceutical composition for use according to the present disclosure comprises the dry extract of flower material of mesua ferrea, wherein the dry extract of flower material of mesua ferrea contains 10 wt.-% to 20 wt.-% of gallotannin as active agent.

In another further preferred embodiment, the pharmaceutical composition for use according to the present disclosure comprises the dry extract of flower material of mesua ferrea, wherein the dry extract of flower material of mesua ferrea contains 20 wt.-% of gallotannin as active agent.

All preferred quantities of extract of flower material of mesua ferrea in an amount of from 60 to 450 mg may have a certain amount of active ingredient gallotannin between 3 wt.-% to 20 wt.-%.

In a preferred embodiment, the pharmaceutical composition for use comprises a dry extract of flower material of mesua ferrea in an amount of 240 mg, wherein the dry extract contains 3 wt.-% to 20 wt.-% of gallotannin as active agent.

In a further preferred embodiment, the pharmaceutical composition for use comprises a dry extract of flower material of mesua ferrea in an amount of 240 mg, wherein the dry extract contains 10 wt.-% to 20 wt.-% of gallotannin as active agent.

In another further preferred embodiment, the pharmaceutical composition for use comprises a dry extract of flower material of mesua ferrea in an amount of 240 mg, wherein the dry extract contains 20 wt.-% of gallotannin as active agent.

### Fillers and excipients

In pharmaceutical compositions designed as powders in various forms for oral administration, lubricants, anti-caking agents and glidants are essential excipients to ensure industrial scalability and efficient manufacturing processes. Lubricants, such as magnesium stearate, stearic acid, and calcium stearate, may be helpful in reducing friction between the powder and the surfaces of manufacturing equipment, such as tablet punches and dies, or capsule filling machines. This minimizes wear on machinery and ensures uniformity in production.

Glidants or anti-caking agents including colloidal silicon dioxide and talc, are added to improve the flow properties of powders, preventing aggregation and ensuring consistent filling during high-speed production. These excipients are particularly helpful for maintaining batch-to-batch consistency, reducing downtime due to equipment blockages, and optimizing production rates. The precise selection and concentration of lubricants and glidants are critical to achieving the desired balance between mechanical properties, such as tablet hardness, and dissolution performance, ensuring that the final product meets both regulatory and therapeutic standards.

Further, optional fillers, such as microcrystalline cellulose, maltodextrin, lactose, or mannitol, may be included to provide bulk and improve compressibility in tablet formulations. Excipients such as binders (e.g., povidone or hydroxypropyl methylcellulose) are used to enhance cohesion, while disintegrants (e.g., sodium croscarmellose or sodium starch glycolate) facilitate the breakdown of the composition in the gastrointestinal tract for improved drug release. Coating or flavoring agents may be incorporated for palatability and ease of ingestion, while pH modifiers and stabilizers can enhance drug solubility and shelf life. These optional components are carefully selected to meet regulatory standards, ensuring the composition's safety, efficacy, and manufacturability.

In a preferred embodiment, the pharmaceutical composition for use according to the present disclosure comprises at least one filler and/or at least one excipient. The at least one filler and/or excipient may be microcrystalline cellulose, lactose, mannitol, maltodextrin, dicalcium phosphate, povidone, hydroxypropyl methylcellulose (HPMC), pregelatinized starch, croscarmellose sodium, sodium starch glycolate, crospovidone, magnesium stearate, stearic acid, colloidal silicon dioxide, talc, citric acid, calcium carbonate, sorbitol, or aspartame.

In a further preferred embodiment, the pharmaceutical composition for use according to the present disclosure comprises at least one filler and/or at least one excipient, preferably selected from the group consisting of magnesium stearate, silicium dioxide, maltodextrin and microcrystalline cellulose.

In another preferred embodiment, the pharmaceutical composition for use according to the present disclosure comprises at least one filler that is present in an amount of from 0.1 wt.-% to 0.5 wt.-%, preferably of from 0.15 wt.-% to 0.30 wt.-% and further preferably of around 0.22 wt.-%. The amount refers to the total weight of the pharmaceutical composition for use.

In a particular preferred embodiment, the pharmaceutical composition for use according to the present disclosure comprises at least one filler, wherein the filler is maltodextrin.

In another preferred embodiment, the pharmaceutical composition for use according to the present disclosure comprises at least one excipient that is present in an amount of from 0.10 wt.-% to 2.0 wt.-%, preferably of from 0.70 wt.-% to 1.50 wt.-% and further preferably of around 1.11 wt.-%. The proportion refers to the total weight of the pharmaceutical composition for use.

In a particular preferred embodiment, the pharmaceutical composition for use according to the present disclosure comprises at least one excipient, wherein the excipient is silicon dioxide and/or magnesium stearate.

### Method of producing

In a second aspect, the present disclosure is directed to a method of producing a pharmaceutical composition according to any one of the preceding claims, wherein the method comprises the steps of:
i. extracting each plant material of cichorium intybus, vitis vinifera, berberis aristate, hamamelis virginiana, azadirachta indica, aesculus hippocastanum and mesua ferrea with an aqueous or hydroalcoholic solvent;
ii. drying of each extract to obtain a powder extract;
iii. providing corresponding amounts of each dried powder extract to obtain the pharmaceutical composition;
iv. optionally blending the pharmaceutical composition, and optionally adding fillers and/or excipients to the pharmaceutical composition prior to blending, to obtain a uniformly distributed powder blend of the pharmaceutical composition.

### Extraction

The disclosed method comprises an extraction of bioactive compounds from plant material using a maceration process in step i., followed by subsequent processing to obtain a pharmaceutical-grade dried extract in step ii. The method includes the steps of preparing plant material by drying and comminating it to increase the surface area for extraction. Methods to optimize the plant material's surface area may include cutting, grinding, slicing, milling, freeze-drying, crushing or shredding that facilitate improved interaction with the extraction solvent and maximizing the yield of bioactive constituents. The method according to the present disclosure is however not limited to these methods and may use any method that is suitable to maximize yield of the extraction.

In a preferred embodiment, the temperature during the optimization of the plant material's surface area may not exceed 35 °C.

The plant material is then immersed in a solvent, preferably an aqueous or a hydroalcoholic solution, at a predetermined ratio, and allowed to macerate under ambient or controlled temperatures for a duration sufficient to extract the desired bioactive compounds. Thereby the plant material should be fully covered by solvent.

In a preferred embodiment, the method of the present disclosure comprises step i., wherein the aqueous solvent is water, preferably water that is purified in a food and/or pharmaceutical grade.

In another preferred embodiment, the method of the present disclosure comprises step i., wherein the solvent is hydroalcoholic.

Hydroalcoholic solvents are composed of a mixture of water and alcohol in varying proportions. Hydroalcoholic solvents are used to extract bioactive compounds from plants for pharmaceutical compositions. This solvent system is highly effective in solubilizing both polar and non-polar phytochemicals, such as alkaloids, flavonoids, terpenoids, and polyphenols, ensuring a broad-spectrum extraction. The alcoholic proportion enhances the dissolution of organic compounds, while water facilitates the extraction of hydrophilic components. The ratio of water to alcohol is carefully optimized based on the target compounds and plant material to be extracted.

In another preferred embodiment, the method of the present disclosure comprises step i., wherein the solvent is hydroalcoholic and contains 50 to 96 % (v/v) of alcohol. The aqueous proportion is thereby water, preferably water that is purified in a food and/or pharmaceutical grade. The alcoholic proportion may be selected from any alcohol that is suitable for the extraction from the plant material that is comprised by the pharmaceutical composition of the present disclosure.

For pharmaceutical compositions, ethanol is the most commonly used organic alcoholic solvent due to its effectiveness, safety profile, and regulatory approval for pharmaceutical and medicinal use. Further optional alternatives may be methanol, isopropanol, butanol or propylene glycol.

In a particular preferred embodiment, the method of the present disclosure comprises step i., wherein the solvent is hydroalcoholic and contains 50 to 96 % (v/v) of ethanol.

In another particular preferred embodiment, the method of the present disclosure comprises step i., wherein the solvent is hydroalcoholic and contains 60, 70, 80, 90 or 96 % (v/v) of ethanol.

The solvent-to-material ratio should be optimized to maximize yield without causing excessive dilution.

In a preferred embodiment, the solvent-to-material ratio is approximately 5 : 1, wherein the amount of solvent is assumed to be determined in Liters and the material is weighted in kilograms.

The extraction vessel must allow for adequate contact time between the plant material and the solvent, with occasional agitation, shaking or stirring to enhance diffusion. Temperature control is essential to prevent degradation of heat-sensitive compounds, and the process may be conducted under ambient or controlled low temperatures, depending on the nature of the extract.

In a preferred embodiment mixture containing solvent and plant material is kept at room temperature for a time period between 10 to 20 days, preferably 12 to 18 days, further preferably 14 or 15 days.

Following the maceration step, the solvent containing the extracted constituents (the menstruum) is separated from the residual plant material (the marc) by filtration or decantation. Optionally, the marc may be squeezed to increase the yield. The resulting filtrate is subjected to concentration using techniques such as rotary evaporation under reduced pressure to remove excess solvent, yielding a concentrated extract. Optionally, purification steps, such as liquid-liquid partitioning or chromatographic separation, may be employed to isolate specific bioactive fractions.

### Drying

The concentrated or purified extract is further processed into a solid form, typically by spray drying, freeze-drying, or vacuum drying, to produce a dry powdered extract suitable for incorporation into pharmaceutical compositions.

In a preferred embodiment, the method for producing the pharmaceutical composition according to the present disclosure comprises a step ii., wherein each extract is dried using a method of vacuum drying to obtain a dried powder extract.

In further preferred embodiment, the method for producing the pharmaceutical composition according to the present disclosure comprises a step ii., wherein each extract is dried using a vacuum tray dryer to obtain a dried powder extract.

Vacuum drying accelerates the drying process by lowering the boiling point of solvents such as water or ethanol, enhancing overall efficiency. The vacuum environment minimizes oxidation, protecting sensitive compounds, and ensures uniform drying across the extract, reducing the risk of inconsistencies. Additionally, vacuum drying helps retain aromatic and volatile compounds, such as essential oils, which are crucial for the therapeutic properties of many plant extracts. It also consumes less energy compared to conventional drying methods, contributing to cost savings, while the enclosed environment reduces the risk of contamination. Lastly, vacuum drying reduces the moisture content of the extract, improving stability, increasing shelf life, and ensuring a cleaner, more controlled production process.

A vacuum tray dryer offers several advantages as a drying method for plant extracts, particularly for preserving sensitive bioactive compounds. By operating under reduced pressure, it also allows for drying at lower temperatures, which helps prevent the thermal degradation of active ingredients, maintaining their full pharmacological potency. This makes vacuum tray drying an optimal method for producing high-quality, stable, and potent plant extracts for pharmaceutical applications.

In another preferred embodiment, the method for producing the pharmaceutical composition according to the present disclosure comprises a step ii., wherein each extract is dried using a method of vacuum drying, wherein a temperature of 35 °C is not exceeded.

In another preferred embodiment, the method for producing the pharmaceutical composition according to the present disclosure comprises a step ii., wherein each extract is dried using a method of vacuum drying, wherein the drying is operated at a pressure of from 35 to 45 mmHg, preferably of from 38 to 43 mmHg, further preferably at 41 mmHg.

In a further preferred embodiment, the method for producing the pharmaceutical composition according to the present disclosure comprises a step ii., wherein the dried extract has a water content below 7 wt.-%, preferably below 6 wt.-%, further preferably below 5 wt.-% after step ii.

The present disclosure is in particular not limited to specific methods for drying each extract, any method that is suitable to obtain a dried extract that subsequently can be processed in a pharmaceutical composition according to the present disclosure may be chosen.

### Amounts of ingredients

The pharmaceutical composition is composed of the seven different extracts as described in step i. To this purpose, it consists of various corresponding amounts of the aforementioned dried extracts provided in step iii. of the method of producing according to the present disclosure:
In a preferred embodiment, the method of producing a pharmaceutical composition for use according to the present disclosure comprises step iii., wherein a dry extract of the root of cichorium intybus is provided in an amount of from 30 to 90 mg, preferably in an amount of from 45 to 70 mg, further preferably in an amount of from 55 to 60 mg, most preferably in an amount of 60 mg.

In a preferred embodiment, the method of producing a pharmaceutical composition for use according to the present disclosure comprises step iii., wherein a dry extract of the leaves of vitis vinifera is provided in an amount of from 40 to 200 mg, preferably in an amount of from 80 to 150 mg, further preferably in an amount of from 100 to 130 mg, most preferably in an amount of 120 mg.

In a preferred embodiment, the method of producing a pharmaceutical composition for use according to the present disclosure comprises step iii., wherein a dry extract of the root and bark of berberis aristate is provided in an amount of from 65 to 500 mg, preferably in an amount of from 150 to 400 mg, further preferably in an amount of from 280 to 350 mg, most preferably in an amount of 320 mg.

In a preferred embodiment, the method of producing a pharmaceutical composition for use according to the present disclosure comprises step iii., wherein a dry extract of the root and bark of berberis aristate is provided in an amount of from 65 to 500 mg, preferably in an amount of from 150 to 400 mg, further preferably in an amount of from 280 to 350 mg, most preferably in an amount of 320 mg.

In a preferred embodiment, the method of producing a pharmaceutical composition for use according to the present disclosure comprises step iii., wherein a dry extract of the bark of hamamelis virginiana is provided in an amount of from 25 to 130 mg, preferably in an amount of from 50 to 110 mg, further preferably in an amount of from 70 to 90 mg, most preferably in an amount of 80 mg.

In a preferred embodiment, the method of producing a pharmaceutical composition for use according to the present disclosure comprises step iii., wherein a dry extract of the leaves of azadirachta indica is provided in an amount of from 4 to 35 mg, preferably in an amount of from 10 to 30 mg, further preferably in an amount of from 15 to 25 mg, most preferably in an amount of 20 mg.

In a preferred embodiment, the method of producing a pharmaceutical composition for use according to the present disclosure comprises step iii., wherein a dry extract of the seeds of aesculus hippocastanum is provided in an amount of from 15 to 110 mg, preferably in an amount of from 40 to 80 mg, further preferably in an amount of from 50 to 70 mg, most preferably in an amount of 60 mg.

In a preferred embodiment, the method of producing a pharmaceutical composition for use according to the present disclosure comprises step iii., wherein a dry extract of the flower material of mesua ferrea is provided in an amount of from 60 to 450 mg, preferably in an amount of from 100 to 350 mg, further preferably in an amount of from 200 to 280 mg, most preferably in an amount of 240 mg.

The seven herbal extracts in the corresponding amounts of each dried powder extract as outlined above result in the pharmaceutical composition. They may optionally be mixed together and blended to obtain a uniformly distributed powder blend of the pharmaceutical composition.

The pharmaceutical composition obtained by providing the corresponding amounts of each dried powder extract may optionally be mixed together with pharmaceutically acceptable excipients, including fillers, binders, disintegrants, and stabilizers, to formulate oral dosage forms such as tablets or capsules. The optional addition of these excipients will take place prior to the blending of the pharmaceutical composition comprising the pharmaceutical composition as obtained in step iii. Fillers and/or excipients may be added in certain amounts and blended to obtain a uniformly distributed powder blend of the pharmaceutical composition.

In a preferred embodiment, the method of producing a pharmaceutical composition for use according to the present disclosure provides at least one filler in step iv. that is present in an amount of from 0.1 wt.-% to 0.5 wt.-%, preferably of from 0.15 wt.-% to 0.30 wt.-% and further preferably of around 0.22 wt.-%. The amount refers to the total weight of the pharmaceutical composition for use.

In another preferred embodiment, the method of producing a pharmaceutical composition for use according to the present disclosure provides at least one excipient in step iv. that is present in an amount of from 0.10 wt.-% to 2.0 wt.-%, preferably of from 0.70 wt.-% to 1.50 wt.-% and further preferably of around 1.11 wt.-%. The amount refers to the total weight of the pharmaceutical composition for use.

The blending of plant extract amounts into pharmaceutical compositions is a critical step to ensure uniformity and consistency of active ingredients throughout the final product. Several methods can be employed to achieve an even distribution of the extract within the formulation, depending on the form of the composition and the scale of production.

In a preferred embodiment, the method of producing a pharmaceutical composition for use according to the present disclosure comprises step iv., wherein the blending of the amounts is performed for at least 15 minutes using a blender.

In another preferred embodiment, the method of producing a pharmaceutical composition for use according to the present disclosure comprises step iv., wherein the blending of the pharmaceutical composition is performed for 15 minutes, 20 minutes, 25 minutes, 30 minutes, 35 minutes or 40 minutes using a blender.

For powdered extracts, blending can be performed using tumble blenders, such as V-blenders, double cone-blenders or octagonal blenders, which allow for gentle mixing of the extract with excipients such as fillers, binders, and other additives. These blenders facilitate the even distribution of the extract by tumbling the powder in a rotating drum, ensuring a uniform blend without the risk of overheating or degradation.

In a preferred embodiment, the method of producing a pharmaceutical composition for use according to the present disclosure comprises step iv., wherein the blending of the pharmaceutical composition is performed at a rotating speed of from 5 to 30 rpm, preferably of from 7 to 20 rpm, further preferably from 8 to 15 rpm.

In another preferred embodiment, the method of producing a pharmaceutical composition for use according to the present disclosure comprises step iv., wherein the blending of the pharmaceutical composition is performed at a rotating speed of 9, 10, 11 or 12 rpm.

For tablet or capsule formulations, granulation methods such as wet granulation or dry granulation may be used to ensure that the extract is evenly distributed within the excipient matrix. After granulation, the final blend is typically subjected to compression or encapsulation, ensuring that each dosage form contains the intended amount of extract.

For industrial-scale production, the extract can be introduced into the formulation using automated systems designed to ensure uniformity in content and precision in dosing. These processes may optionally include blending, encapsulation, or filling technologies, including batch filling, continuous filling, or micro-dosing techniques, to guarantee that each unit of the pharmaceutical composition contains the appropriate amount of active extract, as determined by quality control standards. These systems often include blender-load cells to monitor the weight of materials during blending, ensuring that the correct amount of extract is incorporated into each batch.

### Provision of an administration form

The pharmaceutical composition according to the method of the present disclosure may be further processed to its form of administration.

For oral administration, suitable forms may include any type of powder formulation, such as tablets, pills, pellets, granules, powder containing capsules, and further liquid formulations. Tablets, any sort of compressed powder dose and capsules are preferred for their precise dosing, convenience, and stability, while liquid formulations may be suitable for patients with swallowing difficulties or specific dosing requirements. Controlled-release or enteric-coated tablets can provide sustained delivery of active ingredients, reducing the frequency of administration and enhancing patient compliance. These forms are particularly useful in addressing the underlying vascular and inflammatory components of hemorrhoidal disease.

In a preferred embodiment, the method of producing a pharmaceutical composition for use includes the provision of a form of administration, wherein the pharmaceutical composition is filled into suitable capsules or compressing the powder blend of the pharmaceutical composition into tablets.

The pharmaceutical composition, in the form of a free-flowing powder may be filled into hard gelatine, hydroxypropyl methylcellulose (HPMC), cellulose, pullan, starch or other suitable capsules using any appropriate encapsulation process. Industrial-scale capsule filling may be carried out using augerbased, vibratory, tamping, or dosage-based encapsulation machines, ensuring precise dosing and uniformity. The composition may be optionally sieved and/or blended with excipients and/or filler such as anticaking-agents and disintegrants as outlined in step iv. to enhance processing efficiency and capsule integrity. The selection of the encapsulation method depends on the physicochemical properties of the composition, ensuring stability, bioavailability, and controlled release, if required. Any suitable method known in the art may be employed to achieve the desired formulation characteristics.

An optional sieving of the extract powder before capsule filling may offer several advantages that enhance the quality, consistency, and efficiency of the final pharmaceutical product. By removing agglomerates and ensuring uniform particle size, sieving improves the flowability of the powder, preventing irregular dosing and ensuring that each capsule contains the precise amount of active ingredient. This process also reduces the risk of clogging or inconsistencies during automated filling, leading to a smoother and more efficient manufacturing process. Additionally, sieving improves the overall purity and homogeneity of the formulation.

The pharmaceutical composition may alternatively be formulated into tablets using direct compression, dry granulation, or wet granulation techniques, depending on the properties of the active ingredient and excipients. The composition may be optionally blended with binders, disintegrants, lubricants, and other suitable excipients as outlined in step iv. to facilitate tablet formation, ensuring mechanical strength, uniformity, and controlled dissolution. The blended material is then compressed into tablets of the desired shape and size using rotary tablet presses, single-punch presses, or any other suitable tableting equipment. If required, the tablets may further be coated for protection, modified release, or taste masking. Any method known to those skilled in the art may be applied to achieve an optimal pharmaceutical formulation according to the disclosure.

In a preferred embodiment, the method of producing a pharmaceutical composition for use according to the present disclosure, wherein the composition is filled into a capsule comprising 118.5 to 760 mg, preferably 200 to 600 mg, further preferably 350 to 500 mg and most preferably 450 mg of the pharmaceutical composition.

The minimum amount of a daily dosage of the pharmaceutical composition according to the present disclosure thereby refers to 239 mg, whereas the maximum amount of a daily dosage of the pharmaceutical composition according to the present disclosure refers to 1520 mg of the pharmaceutical composition. The daily dosage is typically administered in two capsules each containing a minimum of 118.5 mg and a maximum of 760 mg.

### Capsule

In a third aspect, the present disclosure is directed to a capsule comprising the pharmaceutical composition for use according to the present disclosure, wherein the capsule comprises 118.5 to 760 mg, preferably 200 to 600 mg, further preferably 350 to 500 mg and most preferably 450 mg of the pharmaceutical composition.

Capsules used to encapsulate a pharmaceutical composition may be composed of gelatine, hydroxypropyl methylcellulose (HPMC), pullulan, starch, starch-based polymers, polyvinyl acetate phthalate (PVAP), hydroxypropyl cellulose (HPC), alginate-based materials, carrageenan-based materials, chitosan-based polymers, pectin-based materials, hypromellose acetate succinate (HPMCAS), and enteric-coated gelatin or HPMC or other suitable biocompatible materials. The material is depending on the desired formulation characteristics, including stability, release profile, and dietary considerations. Hard or soft capsules may be selected based on the physical state of the composition, with hard capsules being suitable for powders, granules, or pellets, while soft gelatine capsules are commonly used for liquid or semi-solid formulations.

Capsule sizes may vary, typically ranging from Size 000 (largest) to Size 5 (smallest), with the appropriate size chosen to accommodate the required dosage while ensuring ease of swallowing. Additional functional coatings may be applied to modify release properties, such as enteric coatings for delayed release or gastric-resistant coatings for targeted delivery. Any suitable encapsulation material and size may be selected based on the formulation requirements and intended therapeutic application.

In a preferred embodiment, the capsule according to the present disclosure is made from cellulose, gelatine, pullulan, starch or starch-based polymers.

In another preferred embodiment, the capsule according to the present disclosure has size 0 containing 118.5 to 760 mg, preferably 200 to 600 mg, further preferably 350 to 500 mg and most preferably 450 mg pharmaceutical composition according to the present disclosure.

In another preferred embodiment, the daily dose is 400 to 1200 mg of the pharmaceutical composition according to the present disclosure.

In another preferred embodiment, the daily dose of 239 to 1520 mg is preferably administered in two capsules each containing 118.5 to 760 mg of the pharmaceutical composition according to the present disclosure, preferably 200 to 600 mg, further preferably 350 to 500 mg and most preferably 450 mg of the pharmaceutical composition.

### Definitions

Ther term 'hydroalcoholic solvent' refers to mixtures of purified water and an alcohol meeting the purity standards of a food-grade or pharmaceutical grade solvent, preferably ethanol is used for the alcohol proportion.

The term 'aqueous' in context of extraction refers to the usage of purified water meeting the purity standards of a food-grade or pharmaceutical grade solvent.

The term 'room temperature' means a temperature of 25±2°C.

The term 'daily dose' refers to an amount of from 239 to 760 mg per day, preferably administered in two capsules containing an equal amount.

### Experimental part

### General Formulation and Preparation

An extract of each herb of the pharmaceutical composition of the present disclosure is prepared in standard manner:

After selecting dried plant material of high-quality, the plant material is firstly grinded. Grinding to medium-fine consistency, the surface area of the plant material is increased, and subsequent extraction process is enhanced. During grinding a temperature of 35 °C should not be exceeded.

The extraction procedure is carried out in a standard maceration process, using either aqueous or a suitable hydroalcoholic solvent for extraction. The plant material is therefore placed in a clean, tightly sealed container and filled up with a suitable solvent for extraction to fully cover and saturate the plant material (approximated ratio of 1 kg plant material : 5 L solvent). Subsequently, the mixture is thoroughly mixed to obtain an even saturation and permeation of the plant material with solvent.

The sealed container is then kept at room temperature for 14 days. During that time, the mixture in the sealed container is stirred or shaken once a day ensuring uniform extraction and solvent circulation.

Once the maceration process in the sealed container is complete, the solid components are separated from the liquid components by filtration and then strained to extract the maximum amount of liquid.

The extract is dried in a standard manner using a vacuum tray dryer to subsequently obtain a powder extract. Drying was performed with a Stokes 338F-4 vacuum oven at a lowered internal pressure of 41 mmHg at a maximum temperature of 35 °C. The remaining water content in the extracts after drying is below 6 %. The individual extracts of the herbs are listed below with the contents of their active ingredient components. These extracts used for the formulation according to the present disclosure have an active ingredient content in the ranges as presented below.

### Cichorium intybus (radix):

Root material of the cichorium intybus plant, commonly known as common chicory, is extracted using water as solvent. The resulting dry extract contains 45 % to 90 % of the active ingredient inulin.

### Vitis vinifera (folium):

Leaf material of the vitis vinifera plant, commonly known as grape wine is extracted using hydro alcohol (90 % ethanol (v/v)) as solvent. The resulting dry extract contains 30 % to 95 % of oligomeric proanthocyanidins as active ingredient.

### Berberis aristate (cortex/radix):

Root and bark material of the berberis aristate plant, commonly known as indian barberry or tree turmeric, is extracted using water as solvent. The resulting dry extract contains 0.4 % to 2 % of the active ingredient berberine.

### Hamamelis virginiana (cortex):

Bark material of the hamamelis virginiana plant, commonly known as witch hazel, is extracted using hydro alcohol (60 % ethanol (v/v)) as solvent. The resulting dry extract contains 3 % to 10 % of hydrolysable tannins as active ingredient.

### Azadirachta indica (folium):

Leaf material of the azadirachta indica plant, commonly known as neem tree, is extracted using hydro alcohol (60 % ethanol (v/v)) as solvent. The resulting dry extract contains 0.2 % to 1 % of the active ingredient nimbidin.

### Aesculus hippocastanum (semen):

Seed material of the Aesculus hippocastanum plant, commonly known as horse chestnut or conker tree, is extracted using hydro alcohol (60 % ethanol (v/v)) as solvent. The resulting dry extract contains 5 % to 20 % of the active ingredient escin.

### Mesua ferrea (flos):

Flower material of the Mesua ferrea plant, commonly known as Nagkesar or Ceylon ironwood, is extracted using a hydro alcohol (60 % ethanol (v/v)) as solvent. The resulting dry extract contains 5 % to 20 % of the active ingredient gallotannin.

Each powdered extract is weighted accurately in a specific amount to obtain the pharmaceutical composition according to the present disclosure. Subsequently, all dry extract amounts are optionally blended to obtain a uniform distributed pharmaceutical composition. Optionally, fillers and excipients, such as magnesium stearate, silicium dioxide, maltodextrin or microcrystalline cellulose can be added prior to this blending process. The resulting uniform blend is sieved and may be encapsulated in suitable capsules, such as cellulose capsule shells, or compressed into tablets afterwards.

### Oral Capsule Formulation and Dosage

To obtain an oral capsule formulation according to the present disclosure, the dry powdered extracts containing active ingredients in the corresponding amounts as described for the general formula are weighed to obtain the pharmaceutical composition. Optionally, excipients and fillers are added to the pharmaceutical composition comprising the dry powdered extracts. The pharmaceutical composition is optionally blended in a suitable mixer for at least 15 minutes to obtain a uniform distributed powder blend of the pharmaceutical composition. Subsequently, the pharmaceutical composition or the optionally powder blend of the pharmaceutical composition is filled into a suitable capsule, after optionally sieving of the powder.

Two capsules with each an amount between 118.5 to 760 mg, refer to a suitable daily dose. The contained amounts of extract for a daily dose according to the present disclosure are displayed in Table 1.

The amounts of a suitable daily dose as displayed in Table 1 are administered on an empty stomach at a minimum of 30 minutes before food consumption over a recommended period of twelve consecutive days.

**Table 1 Ingredients and amounts in an oral capsule formulation.**

| | **Plant name (Latin)** | **Plant part** | **Active substance** | **Active substance** | **Amount of extract in Daily Dose [mg]** |
|---|---|---|---|---|---|
| **1** | cichorium intybus | root | inulin | 45 - 90 wt.-% | 30 - 90 |
| **2** | vitis vinifera | leaf | oligomeric proanthocyanidins | 30 - 95 wt.-% | 40 - 200 |
| **3** | berberis aristate | bark / root | berberine | 0,4 - 2 wt.-% | 65 - 500 |
| **4** | hamamelis virginiana | bark | hydrolyzable tannins | 3 - 10 wt.-% | 25 - 130 |
| **5** | azadirachta indica | leaf | nimbidin | 0,2 - 1 wt.-% | 4 - 35 |
| **6** | aesculus hippocastanum | seed | escin | 5 - 20 wt.-% | 15 - 110 |
| **7** | mesua ferrea | flower | gallotannin | 5 - 20 wt.-% | 60 - 450 |

### Example 1:

The amounts of dry powdered extracts of the seven herbs containing different active ingredients in ratios as indicated in Table 2 are prepared according to the general procedure for formulation and preparation. The amounts of extract (Table 2) resulting in a pharmaceutical composition are each filled into two capsules (Cellulose, size 0, 450 mg total amount in each capsule) referring to a daily dose. The filling of the amount per each capsule of the two capsules is exactly 50 % of the amounts for each extract as indicated in Table 2 (e.g. 60 mg daily dose cichorium intybus extract refers to 30 mg in each of the two capsules).

**Table 2 Ingredients and amounts in a daily dose oral capsule formulation according to Example 1.**

| | **Plant name (Latin)** | **Plant part** | **Active substance** | **Active substance** | **Amount of extract in Daily Dose [mg]** |
|---|---|---|---|---|---|
| **1** | cichorium intybus | root | inulin | 90 wt.-% | 60 |
| **2** | vitis vinifera | leaf | oligomeric proanthocyanidins | 95 wt.-% | 120 |
| **3** | berberis aristate | bark / root | berberine | 2 wt.-% | 320 |
| **4** | hamamelis virginiana | bark | hydrolyzable tannins | 10 wt.-% | 80 |
| **5** | azadirachta indica | leaf | nimbidin | 1 wt.-% | 20 |
| **6** | aesculus hippocastanum | seed | escin | 20 wt.-% | 60 |
| **7** | mesua ferrea | flower | gallotannin | 20 wt.-% | 240 |
| | | | | **Total [mg]:** | **900** |

### Example 2:

The amounts of dry powdered extracts of the seven herbs containing different active ingredients in ratios as indicated in Table 2, are mixed together to obtain a pharmaceutical composition. Furthermore, the filler maltodextrin (0.22 wt.-% of the total weight extract, corresponding to 1 mg per capsule containing 450 mg extract), the excipient magnesium stearate (1.11 wt.-% of the total weight extract, corresponding to 5 mg per capsule containing 450 mg extract) and silicon dioxide (1.11 wt.-% of the total weight extract, corresponding to 5 mg per capsule containing 450 mg extract) as anti-caking agents are added to the pharmaceutical composition.

The pharmaceutical composition is blended for 25 minutes at 10 rpm using a PerMix PDC-200 double cone blender to result in a uniform distributed powder blend. The blended pharmaceutical composition is sieved and 900 mg are filled into two capsules (Cellulose, size 0, exactly 450 mg each capsule) constituting a daily dose.

### Evidence of medical efficacy

The efficacy of the formulation according to the present disclosure for treating hemorrhoidal disease and/or symptoms associated with hemorrhoidal disease was evaluated in medicinal evidence on different patients with ano-rectal conditions grade I to III. Two capsules prepared in accordance to example 1 were administered in the morning with water on an empty stomach at least 30 minutes before food consumption over a period of 10 - 12 consecutive days.

### Example 3:

The patient is a 48-year-old healthy male reporting a long-standing history of anal pruritus. On physical examination, he was found to have grade I hemorrhoids and mild faecal smearing on perianal skin. Recent colonoscopy and laboratory work ordered by the primary care provider were normal. He was counselled on common inciting agents and local irritants and was advised on hygiene, diet modification, and stool-bulking agents. The colorectal surgeon recommended that the patient keep a journal about his symptoms, foods, and household chemicals used. A short-course trial of topical steroid, barrier cream, and topical tacrolimus was not helpful to reduce his symptoms.

Given the persistence of symptoms, the patient started a treatment with a formulation according to the present disclosure. After 12 days of treatment the patient reported a complete resolution of symptoms.

### Example 4:

The patient is a 42-year-old female presented who experienced the passage of a mass (abnormal growth or lump of tissue) from the anus. The clinical history of the patient revealed that she had intermittent recital bleeding for 3 months and a painful mass protruding out of the anus during strenuous work and defecation for 1 month. Initially this mass receded by itself but for the last two weeks the patient had to manipulate it to reposition it. The patient was diagnosed as a case of prolapsed hemorrhoids (grade III). Initially, a combination of local (barrier cream) and oral therapy (micronized purified flavonoids) have been recommended, as well as was advised on hygiene, diet modification, and stool-bulking agents. After 15 days of therapy a persistence of symptoms has been reported.

Afterwards the treatment was continued with a formulation according to the present disclosure. After 10 days of treatment, the patient reported an improvement in all symptoms, especially in pain, discomfort and bleeding. The protruding mass has decreased significantly and there was no need for reposition.

### Example 5:

The patient is a 56-year-old woman with a 3-month history of loose stools associated with intermittent bright red rectal bleeding. The patient also has a history of obesity with special emphasize of abdominal obesity. Proctoscopy confirmed internal hemorrhoids (grade II). She was recommended with hygiene, dietary and lifestyle modification and to keep a record about her symptoms and foods. An oral therapy (micronized purified flavonoids) has been recommended for the following two weeks. A control visit revealed the persistence of symptoms, and it was decided to start a treatment with a formulation according to the present disclosure. After more than 10 days of treatment patient reported complete resolution of symptoms.

### Example 6:

An 80-year-old male following a history of perianal irritation and pruritus is presented. The patient had previously been investigated for iron-deficiency anaemia and the ensuing endoscopy had demonstrated the presence of hemorrhoids. On examination three internal hemorrhoids (grade II) were present. The patient's significant medical history included controlled type 2 diabetes, hypertension and a coronary artery bypass and a valve replacement surgery. The patient was an ex-smoker and did not drink any alcohol. For initial therapy a combination of local (barrier cream) and oral therapy (micronized purified flavonoids) have been recommended, as well as advisory on dietary and lifestyle modification. A persistence of symptoms has been reported after two weeks.

Afterwards, a treatment with a formulation according to the present disclosure was started. After 12 days of continuous treatment with the formulation, the patient reported complete resolution of symptoms after 15 days.

### Example 7:

Patient is a 64-year-old male with more than two months history of pain, discomfort and rectal bleeding. He had a history of obesity, type 2 diabetes and hypertension. During this period, the patient had attempted various over-the-counter creams with limited success. Upon initial per rectal examination, grade II hemorrhoids and mild faecal smearing on the perianal skin were noted. Physical examination confirmed the presence of grade II hemorrhoids with mild faecal soiling on the perianal area. The patient was advised to undergo a short-course trial of topical steroids and barrier cream, in combination with oral micronized purified flavonoids. Additionally, recommendations were made regarding hygiene, dietary modifications, and the use of stool-bulking agents. However, the trial of topical steroids, barrier cream, and topical tacrolimus showed no improvement, and the symptoms persisted.

A new treatment regimen involving a formulation according to the present disclosure was recommended. After more than 10 days of treatment, the patient reported complete resolution of symptoms.

### Example 8:

A 34-year-old female is presented with a history of intermittent bright red rectal bleeding, accompanied by pain, discomfort, itching, and swelling. The patient had given birth six months ago and had a history of obesity. Proctoscopy confirmed the presence of internal hemorrhoids (grade II). The patient was advised to implement hygiene, dietary, and lifestyle modifications, as well as to maintain a record of her symptoms and food intake. A combination of local therapy (barrier cream) and oral treatment (micronized purified flavonoids) was recommended for the next two weeks. The patient reported persistence of symptoms after this period.

As a result, a novel treatment regimen involving a formulation according to the present disclosure was introduced. A further follow-up appointment was scheduled 15 days later, at which the patient reported over 10 days of continuous treatment with the formulation. The patient experienced complete resolution of symptoms following the treatment.

### Example 9:

A 48-year-old male patient is presented with a three-week history of worsening hemorrhoidal symptoms. His clinical history indicated the development of a painful mass protruding from the anus during defecation, which had persisted for four days. Additional symptoms included discomfort and itching. Initially, the mass receded spontaneously, but in recent days, the patient required manual manipulation to reposition it. Rectal examination confirmed grade III hemorrhoids. A combination of local therapy (barrier cream) and oral treatment (micronized purified flavonoids) was recommended, along with advice on hygiene, dietary modifications, and the use of stool-bulking agents. The patient reported mild improvement in symptoms after 15 days.

Given the persistence of symptoms, a continued treatment with a formulation according to the present disclosure was introduced. The patient confirmed more than 10 days of continuous treatment. The treatment resulted in significant improvement across all symptoms, with a marked reduction in the size of the protruding mass after 15 days.

### Embodiments

The present disclosure also pertains to the following numbered embodiments.
1. A pharmaceutical composition for use in treating a hemorrhoidal disease and/or the symptoms associated with a hemorrhoidal disease, wherein the composition comprises:
   a dry extract of the root of cichorium intybus,
   a dry extract of the leaves of vitis vinifera,
   a dry extract of the root and bark of berberis aristate,
   a dry extract of the bark of hamamelis virginiana,
   a dry extract of the leaves of azadirachta indica,
   a dry extract of the seeds of aesculus hippocastanum,
   a dry extract of flower material of mesua ferrea.
2. The pharmaceutical composition for use according to embodiment 1, wherein the composition comprises the dry extract of the root of cichorium intybus in an amount of from 30 to 90 mg, preferably in an amount of from 45 to 75 mg, further preferably in an amount of from 55 to 65 mg, most preferably in an amount of 60 mg.
3. The pharmaceutical composition for use according to any one of the preceding embodiments, wherein the dry extract of the root of cichorium intybus contains 45 wt.-% to 95 wt.-%, preferably 60 to 90 wt.-%, further preferably 90 wt.-% of inulin as active ingredient.
4. The pharmaceutical composition for use according to any one of the preceding embodiments, wherein the composition comprises the dry extract of the leaves of vitis vinifera in an amount of from 40 to 200 mg, preferably in an amount of from 80 to 150 mg, further preferably in an amount of from 100 to 130 mg, most preferably in an amount of 120 mg.
5. The pharmaceutical composition for use according to any one of the preceding embodiments, wherein the dry extract of the leaves of vitis vinifera contains 30 wt.-% to 95 wt.-%, preferably 60 to 95 wt.-%, further preferably 95 wt.-% of oligomeric proanthocyanidins as active ingredient.
6. The pharmaceutical composition for use according to any one of the preceding embodiments, wherein the composition comprises the dry extract of the root and bark of berberis aristate in an amount of from 65 to 500 mg, preferably in an amount of from 150 to 400 mg, further preferably in an amount of from 280 to 350 mg, most preferably in an amount of 320 mg.
7. The pharmaceutical composition for use according to any one of the preceding embodiments, wherein the dry extract of the root and bark of berberis aristate contains 0.4 wt.-% to 7 wt.-%, preferably 1 wt.-% to 3 wt.-%, further preferably 2 wt.-% of berberine as active ingredient.
8. The pharmaceutical composition for use according to any one of the preceding embodiments, wherein the composition comprises the dry extract of the bark of hamamelis virginiana in an amount of from 25 to 130 mg, preferably in an amount of from 50 to 110 mg, further preferably in an amount of from 70 to 90 mg, most preferably in an amount of 80 mg.
9. The pharmaceutical composition for use according to any one of the preceding embodiments, wherein the dry extract of the bark of hamamelis virginiana contains 2 wt.-% to 10 wt.-%, preferably 7 wt.-% to 10 wt.-%, further preferably 10 wt.-% of hydrolysable tannins as active ingredient.
10. The pharmaceutical composition for use according to any one of the preceding embodiments, wherein the composition comprises the dry extract of the leaves of azadirachta indica in an amount of from 4 to 35 mg, preferably in an amount of from 10 to 30 mg, further preferably in an amount of from 15 to 25 mg, most preferably in an amount of 20 mg.
11. The pharmaceutical composition for use according to any one of the preceding embodiments, wherein the dry extract of the leaves of azadirachta indica contains 0.2 wt.-% to 1 wt.-%, preferably 0.5 wt.-% to 1 wt.-%, further preferably 1 wt.-% of nimbidin as active ingredient.
12. The pharmaceutical composition for use according to any one of the preceding embodiments, wherein the composition comprises the dry extract of the seeds of aesculus hippocastanum in an amount of from 15 to 110 mg, preferably in an amount of from 40 to 80 mg, further preferably in an amount of from 50 to 70 mg, most preferably in an amount of 60 mg.
13. The pharmaceutical composition for use according to any one of the preceding embodiments, wherein the dry extract of the seeds of aesculus hippocastanum contains 5 wt.-% to 20 wt.-%, preferably 10 wt.-% to 20 wt.-%, further preferably 20 wt.-% of escin as active ingredient.
14. The pharmaceutical composition for use according to any one of the preceding embodiments, wherein the composition comprises the dry extract of flower material of mesua ferrea in an amount of from 60 to 450 mg, preferably in an amount of from 100 to 350 mg, further preferably in an amount of from 200 to 280 mg, most preferably in an amount of 240 mg.
15. The pharmaceutical composition for use according to any one of the preceding embodiments, wherein the dry extract of flower material of mesua ferrea contains 3 wt.-% to 20 wt.-%, preferably 10 wt.-% to 20 wt.-%, further preferably 20 wt.-% of gallotannin as active agent.
16. The pharmaceutical composition for use according to any one of the preceding embodiments, wherein the composition additionally comprises at least one filler and/or at least one excipient, preferably selected from the group consisting of magnesium stearate, silicium dioxide, maltodextrin and microcrystalline cellulose.
17. The pharmaceutical composition for use according to any one of the preceding embodiments, wherein the symptoms associated with the hemorrhoidal disease are selected from the group consisting of itching in the anal region, irritation in the anal region, swelling near the anus, inflammation, mucosal ulceration, ischemia, thrombosis, external hemorrhoids, rectal bleeding, protrusion, pain and discomfort.
18. The pharmaceutical composition for use according to any one of the preceding embodiments, wherein the pharmaceutical compositions can take the form of tablets, pills, pellets, powders, granules, capsules, capsules containing powders, or any other form suitable for use, preferably wherein composition is in the form of a tablet or of a capsule.
19. The pharmaceutical composition for use according to any one of the preceding embodiments, wherein the method of administration includes oral administration.
20. A method of producing a pharmaceutical composition according to any one of the preceding embodiments, wherein the method comprises the steps of:
   (i) extracting each plant material of cichorium intybus, vitis vinifera, berberis aristate, hamamelis virginiana, azadirachta indica, aesculus hippocastanum and mesua ferrea with an aqueous or hydroalcoholic solvent;
   (ii) drying of each extract to obtain a dried powder extract;
   (iii) providing corresponding amounts of each dried powder extract to obtain the pharmaceutical composition;
   (iv)optionally blending the pharmaceutical composition, and optionally adding fillers and/or excipients to the pharmaceutical composition prior to blending, to obtain a uniformly distributed powder blend of the pharmaceutical composition.
21. The method according to embodiment 20, wherein the pharmaceutical composition is filled into suitable capsules, or the powder blend of the pharmaceutical composition is compressed into tablets.
22. The method according to embodiments 20 or 21, wherein the hydroalcoholic solvent contains 50 to 96 % (v/v) ethanol.
23. The method according to any one of the embodiments 20 to 22, wherein the blending of the proportions in step iv. is performed for at least 15 minutes using a blender.
24. The method according to embodiments 20 to 23, wherein the pharmaceutical composition is filled into a capsule comprising 118.5 to 760 mg, preferably 200 to 600 mg, further preferably 350 to 500 mg and most preferably 450 mg of the composition.
25. A capsule, comprising the pharmaceutical composition for use according to embodiments 1 to 19, wherein the capsule comprises 118.5 to 760 mg, preferably 200 to 600 mg, further preferably 350 to 500 mg and most preferably 450 mg of the pharmaceutical composition.
26. The capsule according to embodiment 25, wherein the capsule is made from cellulose, gelatine, pullulan, starch or starch-based polymers.

## Claims

1. A pharmaceutical composition for use in treating a hemorrhoidal disease and/or the symptoms associated with a hemorrhoidal disease, wherein the composition comprises:
a dry extract of the root of cichorium intybus,
a dry extract of the leaves of vitis vinifera,
a dry extract of the root and bark of berberis aristate,
a dry extract of the bark of hamamelis virginiana,
a dry extract of the leaves of azadirachta indica,
a dry extract of the seeds of aesculus hippocastanum,
a dry extract of flower material of mesua ferrea.

2. The pharmaceutical composition for use according to claim 1, wherein the composition comprises the dry extract of the root of cichorium intybus in an amount of from 30 to 90 mg, preferably in an amount of from 45 to 75 mg, further preferably in an amount of from 55 to 65 mg, most preferably in an amount of 60 mg, and/or wherein the dry extract of the root of cichorium intybus contains 45 wt.-% to 95 wt.-%, preferably 60 to 90 wt.-%, further preferably 90 wt.-% of inulin as active ingredient.

3. The pharmaceutical composition for use according to any one of the preceding claims, wherein the composition comprises the dry extract of the leaves of vitis vinifera in an amount of from 40 to 200 mg, preferably in an amount of from 80 to 150 mg, further preferably in an amount of from 100 to 130 mg, most preferably in an amount of 120 mg, and/or wherein the dry extract of the leaves of vitis vinifera contains 30 wt.-% to 95 wt.-%, preferably 60 to 95 wt.-%, further preferably 95 wt.-% of oligomeric proanthocyanidins as active ingredient.

4. The pharmaceutical composition for use according to any one of the preceding claims, wherein the composition comprises the dry extract of the root and bark of berberis aristate in an amount of from 65 to 500 mg, preferably in an amount of from 150 to 400 mg, further preferably in an amount of from 280 to 350 mg, most preferably in an amount of 320 mg, and/or wherein the dry extract of the root and bark of berberis aristate contains 0.4 wt.-% to 7 wt.-%, preferably 1 wt.-% to 3 wt.-%, further preferably 2 wt.-% of berberine as active ingredient.

5. The pharmaceutical composition for use according to any one of the preceding claims, wherein the composition comprises the dry extract of the bark of hamamelis virginiana in an amount of from 25 to 130 mg, preferably in an amount of from 50 to 110 mg, further preferably in an amount of from 70 to 90 mg, most preferably in an amount of 80 mg, and/or wherein the dry extract of the bark of hamamelis virginiana contains 2 wt.-% to 10 wt.-%, preferably 7 wt.-% to 10 wt.-%, further preferably 10 wt.-% of hydrolysable tannins as active ingredient.

6. The pharmaceutical composition for use according to any one of the preceding claims, wherein the composition comprises the dry extract of the leaves of azadirachta indica in an amount of from 4 to 35 mg, preferably in an amount of from 10 to 30 mg, further preferably in an amount of from 15 to 25 mg, most preferably in an amount of 20 mg, and/or wherein the dry extract of the leaves of azadirachta indica contains 0.2 wt.-% to 1 wt.-%, preferably 0.5 wt.-% to 1 wt.-%, further preferably 1 wt.-% of nimbidin as active ingredient.

7. The pharmaceutical composition for use according to any one of the preceding claims, wherein the composition comprises the dry extract of the seeds of aesculus hippocastanum in an amount of from 15 to 110 mg, preferably in an amount of from 40 to 80 mg, further preferably in an amount of from 50 to 70 mg, most preferably in an amount of 60 mg, and/or wherein the dry extract of the seeds of aesculus hippocastanum contains 5 wt.-% to 20 wt.-%, preferably 10 wt.-% to 20 wt.-%, further preferably 20 wt.-% of escin as active ingredient.

8. The pharmaceutical composition for use according to any one of the preceding claims, wherein the composition comprises the dry extract of flower material of mesua ferrea in an amount of from 60 to 450 mg, preferably in an amount of from 100 to 350 mg, further preferably in an amount of from 200 to 280 mg, most preferably in an amount of 240 mg, and/or wherein the dry extract of flower material of mesua ferrea contains 3 wt.-% to 20 wt.-%, preferably 10 wt.-% to 20 wt.-%, further preferably 20 wt.-% of gallotannin as active agent.

9. The pharmaceutical composition for use according to any one of the preceding claims, wherein the composition additionally comprises at least one filler and/or at least one excipient, preferably selected from the group consisting of magnesium stearate, silicium dioxide, maltodextrin and microcrystalline cellulose.

10. The pharmaceutical composition for use according to any one of the preceding claims, wherein the symptoms associated with the hemorrhoidal disease are selected from the group consisting of itching in the anal region, irritation in the anal region, swelling near the anus, inflammation, mucosal ulceration, ischemia, thrombosis, external hemorrhoids, rectal bleeding, protrusion, pain and discomfort.

11. The pharmaceutical composition for use according to any one of the preceding claims, wherein the method of administration includes oral administration; and wherein the pharmaceutical compositions can take the form of tablets, pills, pellets, powders, granules, capsules, capsules containing powders, or any other form suitable for use, preferably wherein composition is in the form of a tablet or of a capsule.

12. A method of producing a pharmaceutical composition according to any one of the preceding claims, wherein the method comprises the steps of:
i. extracting each plant material of cichorium intybus, vitis vinifera, berberis aristate, hamamelis virginiana, azadirachta indica, aesculus hippocastanum and mesua ferrea with an aqueous or hydroalcoholic solvent;
ii. drying of each extract to obtain a dried powder extract;
iii. providing corresponding amounts of each dried powder extract to obtain the pharmaceutical composition;
iv. optionally blending the pharmaceutical composition, and optionally adding fillers and/or excipients to the pharmaceutical composition prior to blending, to obtain a uniformly distributed powder blend of the pharmaceutical composition.

13. The method according to claim 12, wherein the pharmaceutical composition is filled into suitable capsules, wherein a capsule comprises 118.5 to 760 mg, preferably 200 to 600 mg, further preferably 350 to 500 mg and most preferably 450 mg of the composition; or wherein the powder blend of the pharmaceutical composition is compressed into tablets.

14. The method according to claims 12 or 13, wherein the hydroalcoholic solvent contains 50 to 96 % (v/v) ethanol and/or wherein the blending of the proportions in step iv. is performed for at least 15 minutes using a blender.

15. A capsule comprising the pharmaceutical composition for use according to claims 1 to 11, wherein the capsule is made from cellulose, gelatine, pullulan, starch or starch-based polymers; and/or wherein the capsule comprises 118.5 to 760 mg, preferably 200 to 600 mg, further preferably 350 to 500 mg and most preferably 450 mg of the pharmaceutical composition.
